Europäisches Patentamt

European Patent Office

Office européen des brevets

(19)

(11) Publication number: **0 179 450**
**A2**

(12) **EUROPEAN PATENT APPLICATION**

(21) Application number: **85113410.6**

(22) Date of filing: **22.10.85**

(51) Int. Cl.⁴: **G 01 N 33/53**
**G 01 N 33/577, A 61 K 39/395**
**C 12 N 15/00, A 61 K 39/44**
**A 61 K 49/02, C 12 P 21/00**
**C 07 K 15/14, C 07 K 3/20**
**G 01 N 33/533, G 01 N 33/574**

(30) Priority: **22.10.84 IL 73281**

(43) Date of publication of application:
**30.04.86 Bulletin 86/18**

(84) Designated Contracting States:
**AT BE CH DE FR GB IT LI LU NL SE**

(71) Applicant: **YISSUM RESEARCH AND DEVELOPMENT COMPANY OF THE HEBREW UNIVERSITY OF JERUSALEM**
**46, Jabotinsky Street P.O. box 4279**
**Jerusalem 91042(IL)**

(72) Inventor: **Gazit, Yair**
**9 Sheshet Haiamim Street**
**Mevaseret Zion(IL)**

(74) Representative: **Vossius Vossius Tauchner Heunemann Rauh**
**Siebertstrasse 4 P.O. Box 86 07 67**
**D-8000 München 86(DE)**

(54) Purified antigen isolated from sera of leukemia and lymphoma patients, novel antibodies to said antigen, methods of preparing same and diagnostic and therapeutic methods empolying said antibody.

(57) There are disclosed monoclonal and polyclonal antibodies which react with B cells derived from Burkitt lymphoma (BL) and chronic lymphocitic leukemia (CLL) and do not react with B cells and T cells derived from normal individuals or B cells and T cells derived from patients with cancer disorders other than BL or CL. Also disclosed are a glycoprotein of 70KD in substantially pure form, which reacts with said antibodies, methods for preparing said antibodies and said glycoprotein and diagnostic and therapeutic methods employing said antibodies.

EP 0 179 450 A2

VOSSIUS · VOSSIUS · TAUCHNER · HEUNEMANN · RALH

PATENTANWÄLTE  **0179450**

SIEBERTSTRASSE 4 · 8000 MÜNCHEN 80 · PHONE: (089) 474075
TELECOPIER 089-472001 (GR.IIu.III) · TELEX 5-29 453 VOPAT D

Our Ref.:  U 114EP                          22. Okt. 1985
Case:        5030

YISSUM RESEARCH DEVELOPMENT COMPANY OF THE HEBREW UNIVERSITY
OF JERUSALEM
Jerusalem / Israel

---

Purified antigen isolated from sera of leukemia and lymphoma
patients, novel antibodies to said antigen, methods of preparing
same and diagnostic and therapeutic methods employing said anti-
body

---

The present invention relates to a newly identified leukemia
and lymphoma associated antigen, to polyclonal and monoclonal
antibodies to said antigen, to methods of producing said
antibodies and said antigen, to a hybridoma producing said
monoclonal antibody and to therapeutic and diagnostic methods
employing this antibodies.

Most leukemias and lymphomas are characterized by a maturation arrest at a defined point along the developmental pathway, resulting in a rapid proliferation of lymphoid cells of a given differentiation stage. Accurate characterization of the differentiation stage of leukemia or lymphoma is very important for prognosis and requires accurate definition of the various stages of differentiation along the developmental pathway of lymphoid cells, as outlined by Volger et al. in Prog. in Hemat. 11, 1-45, 1979. Two principal classes of lymphocytes are involved in the immune system of humans and animals. The first of these, (the thymus-derived cells or T cells) is differentiated in the thymus from haemopoietic stem cells. While within the thymus, the differentiating cells are termed "thymocytes." The mature T cells emerge from the thymus and circulate between the tissues, lymphatics, and the bloodestream. These T cells form a large prportion of the pool of circulating small lymphocytes. They have immunological specificity and are directly involved in cell-mediated immune responses (such as graft rejection) as effector cells. Although T cells do not secrete humoral antibodies, they are sometimes required for the secretion of these antibodies by the second class of lymphocytes discussed below. Some types of T cells play a regulating function in other aspects of the immune system. The mechanism of this process of cell cooperation is not yet completely understood. Maturation arrest of these types of cells and unbalanced proliferation are characteristic to T type leukemias.

The second class of lymphocytes (the bone marrow-derived cells or B cells) are those which secrete antibodies. They also develop from haemopoietic stem cells, but their differentiation is not determined by the thymus. In birds, they are differentiated in an organ analogous to the thymus, called the Bursa of Fabricius. In mammals, however, no equivalent organ has been discovered, and it is thought that these B cells differentiate within the bone marrow.

The ability to identify and/or suppress classes or subclasses of B cells is important for diagnosis or treatment of various B-leukemias and lymphomas. For example, certain leukemias and lymphomas have differing prognosis depending upon whether they are of B cell or T cell origin, and upon the degree of maturation of these cells. Thus, evaluation of the prognosis of the disease depends upon distinguishing between classes of lymphomcytes and stage of maturation. See, for example, A.C. Aisenberg and J.C.

Long, in the American Journal of Medicine, 58, 300 (March, 1975);
D. Belpomme et al. in "Immunological Diagnosis of Leukemias and
Lymphomas," S. Thierfelder et al., eds., Springer, Heidelberg,
1977, 33-45; and D. Belpomme et al. in British Journal of
Haematology, 38, 85, 1978.

Several markers for B-cell differentiation were determined as
follows: a hypothetical B-cell progenitor committed to
differentiate along the B-cell pathway gives rise to a pre B-cell,
which is characterized by high rate of DNA synthesis along with
the appearance of cytoplasmic immunoglobulin (cIg) of the IgM or
IgD type, the presence of the so-called B-cell differentiation
marker (BDA) found on all B-cells, and the presence of the DR
antigen of the HLA complex. Acute lymphoblastic leukemia (ALL) is
characteristic to this stage of differentiation. The next defined
stage of differentiation is characterized by the loss of the
proliferative capacity, whereas most other markers are the same.
Chronic lymphocytic leukemia (CLL) and Burkitt's lymphoma (BL) are
related to this stage of differentiation. The next three stages of
development are referred to as maturation steps in which typical
B-cell markers appear, including: a) surface membrane
immunoglobulins (SmIg); b) appearance of receptors for mouse
erythrocytes; c) appearance of full mitogen-antigen response; d) appearance of receptors for Fc protions of IgG; and e) appearance of receptors for $C_3$ component of complement and receptors to Epstein-Barr virus (EBV). Non-Hodgkin's lymphomas are characteristic of this stage of differentiation. The final stage of differentiation is characterized by the appearance of plasma cells and memory cells following stimulation by mitogen or antigen. Later, plaisma cells lose DR antigen, SmIg and some of the Fc receptors and aquire cIg of the IgM/IgG type and produce antibodies. See, for example, T. Godal and S. Funderud in Advances in Cancer Res., 36, 211-255, 1982; and C.M. Balch et al. in J Immunol.123, 2322-2328, 1978.

A lot of research effort has recently been devoted to defining B-cell associated antigens and prepapring antibodies reacting therewith. Greaves at al. in Clin. Immunol. Immunopath. 4, 67-84, 1975, defined an antigen common to most accute lymphoblastic leukemia (CALLA) and to chronic myelogenous leukemia (CML) in the blast crisis. The antigen is a glycoprotein of about 100 KD (molecular weight of 100,000) and monoclonal antibodies reacting with this antigen were prepared by Ritz et al. as described in Nature, 283, 583-585, 1980. Those antibodies do not react with either B-cells or T-cells from peripheral blood, nor with B-cells from tonsils and lymph nodes. However, they do react with cells from bone marrow and with cells from fetal liver. Therefore CALLA is considered an antigen found on early type of pre-B-cells, and it is diagnostically employed as a marker for ALL.

Stachenko et al. in J. Immunol., 125, 1678-1685, 1980 and Nadler et al. in J. Immunol., 126, 1941-1947, 1981 were successful in obtaining monoclonal antibodies $B_1$ and $B_2$ specific for B-cells recognizing normal B-cells of all stages of differentiation except for plasma and memory cells. These antigens of 30 KD ($B_1$) and 140 KD ($B_2$) were found on ALL and CLL type of leukemias and B-cell type lymphomas. Lymphocytes from CML patient were not reactive neither with $B_1$ or $B_2$ antibodies except for blast crisis, as reported by Nadler et al. in Prog. in Haematology 12, 187-225, 1982. Therefore these antibodies can not serve as a tool in diagnosing a specific type of leukemia or lymphoma.

Burkitt's lymphoma (BL) associated antigen (BLA) and antibodies reactive with BL and CLL which has been assigned the number 38.13 were described by Weils et al. in Poc. Natl. Acad. Sci. 78, 6485-6488, 1981. However, Weils et al. (ibid.) found that 38.13 also react with other types of leukemias and lymphomas. Therefore, the clinical value of this monoclonal antibody has not yet been evaluated, and is probably limited, because of its rather broad spectrum of specificity.

Nadler et al. in J. Immunol. 131, 244-250, 1983 have characterized a novel antibody (B4) to an antigen of a molecular weight over 120,000, which reacts with a large spectrum of normal and malignant B-cells including normal peripheral blood B-cells as well as pre-B-All cells. Thus B4 is of no diagnostic value.

LB-1 and BA-1, monoclonal antibodies prepared by Yokochi et al. as described in J. Immunol. 128, 823-827, 1982 and Abramson et al. in J. Immunol. 126, 83-88, 1981 respepctively, were found to be of the "Pan-B" type and therefore of no diagnostic value for their broad recognition of all stages of B-cells differentiation.

Burkitt's lymphoma is a relatively rare form of acute lymphoma occuring mostly in young children. Chronic lymphocytic leukemia, on the other hand, is the most common leukemia and almost half of all known leukemia-lymphoma cases are of CLL type. CLL is a relatively benign form of leukemia which effects mostly people of European origin of over fifty years of age and is characterized by an increase in the number of B-cell lymphocytes, by lymph gland enlargement and splenic enlargement. There may be some weight loss, but patients may often carry on for some years without treatment and without any major symptoms developing. Under treatment the patient can be kept in reasonable good health for many years, the life span in this disease is measured in terms of 5, 10 and even 15 years. Ultimately, however, in most cases transition to prelymphocytic leukemia (PLL) or to acute lymphoblastic leukemia (ALL) occurs and within a few weeks or months death ensues. An early detection of the transition of the disease, followed by an appropriate treatment could prolong the life of patients affected by CLL and ultimately suppress the disease completely.

As stated above, CLL is associated with lymphocytosis, i.e. an increase in white blood cell count to over ten million cells per cubic centimeter from the normal level of 1-2 million cells per cubic centimeter in healthy subjects. However, since lymphocytosis is associated with many other forms of leukemia as well as with other non malignant diseases such as infectious mononucleosis and other viral and bacterial infections, it could merely serve as an indication to a disorder.

The staging of CLL was described by Rai et al. in Blood 46, 219-234, 1975. According to Rai's staging, stage 0 is diagnosed predominantly by a long series of tests such as: lymphocytosis of 10,000 cells/mm and over. However a positive diagnosis of CLL requires additionally the following of surface markers i.e. SmIg, mouse erythrocytes rosetting, and CALLA; the following of cytological marker, i.e. non specific esterase, acid phosphatase and periodic acid schiff stain (PAS); and cell morphology. Each of the above mentioned markers is not specific to CLL and actually most of them are negative to CLL and it is only the combined data obtained from all these tests that can diagnose CLL at stage 0.

The present complexity of diagnosing CLL and the inability to detect it prior to actual complaints by the patient is, of course, a great disadvantage in the early detection and treatment of CLL patients.

It is therefore, the object of the present invention to provide for a direct, one step diagnosis of CLL and BL. The test is based on an immunological reaction between a novel antibody and a newly identified associated antigen present on B-lymphocytes from peripheral blood of CLL and BL patients.

It is accordingly the object of the invention to provide for, the novel antibody against an antigen found on CLL and BL cells and for methods for its preparation. It is also an object of the invention to provide hybridomas which produce antibodies against an antigen found on CLL and BL cells.

It is a further object of the invention to provide for the antigen mentioned above in a substantially pure form and for methods of its isolation and purification.

A further object is to provide for methods of treating CLL and BL patients.

A still further object is to provide for pharmaceutical composition containing the antibody together with a medicament against CLL and BL cells.

Other objects and advantages of the invention will become apparent from the disclosure of the invention.

In satisfaction of the foregoing objects, there is provided by this invention monoclonal and polyclonal antibodies which react with B cells derived from BL and CLL patients and do not react with B cells and T cells derived from normal individuals or B cells and T cells derived from patients with cancer disorders other than BL or CLL. Preferably the antibodies of the invention do not react with peripheral blood lymphocytes, red blood cells, platelets, granulocytes, non-cancerous B-lymphoblastoid cell lines, B or T cells stimulated by lectin mitogen, T-All, AML, CML cell lines, various T-cell lines and cell lines derived from breast, colon, ovary, liver and lung cancer patients. Therefore, the antibodies of the invention are specific to an antigen present in B-cells of BL and CLL cells.

The invention also provides for a glycoprotein of 70 KD hereinafter referred to as GP 70. The GP 70 is isolated and purified from sera of CLL and BL patients. Methods of obtaining GP 70 from sera of BL and CLL patients by series of affinity chromatography separation are also provided by the invention.

Preferably the polyclonal antibodies of the invention are prepared by a method which comprises the following steps:

(a) immunizing animals with GP 70;

(b) bleeding said animals to collect antisera;

(c) absorbing said antisera on tissue capable of binding all normal human antibodies if there are any;

(d) centrifuging the suspension; and

(e) recovering the antibody from the supernatant.

The invention also provides for a novel hybridoma producing a novel monoclonal antibody to an antigen found on CLL and BL. The hybridoma was prepared generally following the method of Milstein and Kohler in Nature 256, 495-497, 1975. Preferably, following immunization of mice with pure GP 70 isolated from serum of BL patients, the spleen cells of the immunized mice were fused with cells from a mouse myeloma lines 653 and the resultant hybridomas screened for those with supernatants containing antibody which gave selective binding to BL patient's lymphocytes. The desired hybridomas were subsequently cloned and characterized. Inter alia, hybridoma (B$_2$) was obtained which produces an

-12-

antibody (anti GP 70) against an antigen on essentially most CLL

and BL cells. Not only does this antibody react with the above mentioned cells, but it also does not react with normal peripheral blood cells. This particular hybridoma ($B_2$) produces anti-

bodies of the IgM type. The hybridoma ($B_2$) which is a preferred embodiment of the present invention, was deposited on with the National Collection of Cultures of Microorganisms (CNCM) of the Pasteur Institute, Paris, France, and was assigned the number

This invention also provides for diagnostic and therapeutic
methods employing the antibodies of the invention and for pharmaceutical compostions containing them.

The antibodies of the invention provide for a method of diagnosis of BL and CLL which comprises reacting serum of patient or other fraction of body fluid of patient with the antibody and determining the immune complex if obtained with a known

immunological test method. Preferably, a method of diagnosis for .

BL and CLL in accordance with the invention comprises of the following steps:

(a)   obtaining a mononuclear fraction of lymphocytes from patient's blood to be tested;

(b)   adding to said fraction the antibody of the invention;

(c)   precipitating said antibody by a second labled antibody directed against the first antibody; and

0179450

-13-

(d)  detecting the labled antibody in the precipitate for a positive result.

Preferably, the second antibody mentioned above is labled by fluorescein. However, other common lables could also be employed.

It should be contemplated that the present invention extends also to other known immunological methods such as RIA (radio immuno assay) and ELISA (Enzyme linked immuno assay) employing the antibodies of the invention for the detection of BL and CLL.

The invention also provides for an in vivo imaging of BL tumor by means of radioactive agent bound to anti GP 70. Common radioactive agents for imaging are applicable e.g. Na I.

The pharmaceutical compositions of the invention comprises as active material a medicament against BL and CLL bound to anti GP 70 together with a pharmaceutically acceptable carrier. Such pharmaceutical compositions are effective in targeting the medicament to the sites containing GP 70 and therefore allow a significant decrease in the amount of active material necessary for treatment. Examples of medicaments for use in the pharmaceutical compositions are 5 fluoro uracil and adriamycin.

The invention also provides for toxin compositions which are effective in targeting ricin toxin bound to anti GP 70 which recognizes and binds to the tumor cells.

In accordance with the invention, there are also provided several methods of treating BL and CLL as follows:

(a)      administering  to a patient an effective  amount of a pharmaceutical  composition  containing  a medicament  directed against BL and CLL bound to anti GP 70.

(b)      administering  to a patient an effective amount of anti GP 70 bound to ricin toxin.

(c)      administering  to a patient an effective amount of anti GP 70 with  no  drug attached to it, for immune killing of cancer cell by complimented mediated lysis.

(d)      removal  of  CLL  and  BL  from plasma by means of techniques related  to  hemoperfusion comprising  subjecting  the  plasma  to selective  binding  of  tumor  cells  to  anti GP 70 bound to solid support.

     The     invention     is   further   illustrated   by   the   following non-limiting examples:

Example 1

Isolation and purification of GP 70 from BL sera

a. Stage I.

     One   hundred   ml   pooled   BL   sera   containing 12g protein are dialysed   against  20 mM phosphate Buffer (PB) at pH 7.8 and passed through a DEAE-52 cellulose column pre equilibrated with same buffer. Unbound material (immunoglobulin fraction) is removed. Bound material is eluted by passing 50 mM PB at pH 7.8.

b. Stage II.

Bound eluted material containing 2 mg protein is passed through a concanavalin A (Con A) sepharose column. Passed material is discarded. Bound material is eluted by passing methyl mannoside (0.1 M in 50 mM PB at pH 7.8).

c. Stage III.

Bound eluted material is passed through a Ricin Communis Agglutinin (RCA) sepharose column and the non-bound material is collected to yield about 0.1mg of homogeneous GP 70.

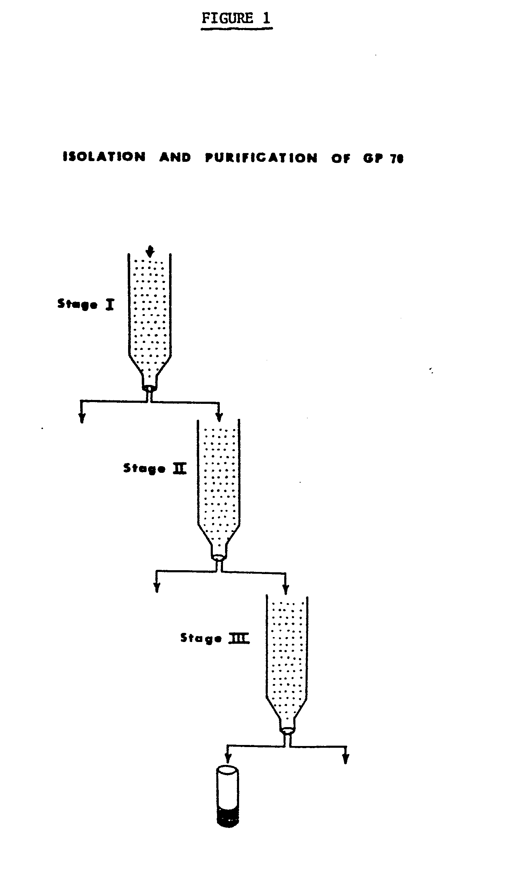

Purification stages I-III are shown schematically in Figure 1.

Example 2

Production of polyclonal anti GP 70

Female New Zeland rabbits (1.5 kg each), are immunized with purified preparation of GP 70 (10 μg/ml in PBS containing complete Freund's Adjuvant; 1:1 v/v). Immunization performed by intramuscular injection followed by subcutaneous injections at 3-4 places. Rabbits are injected in the above mentioned procedure every two weeks and bleeding is performed 10 days post injection. The highest titer of anti GP /0 antibodies is obtained at the fourth bleeding.

Pooled antisera from the fourth bleeding are absorbed on ground powder of lyophilized human spleen slices. Briefly, spleen powder is washed three times with phosphate buffered saline (PBS). Equivolume amount of antiserum or normal rabbit serum (NRS) is added, followed by resuspension and incubation at 37°C with vigorous shaking. Suspension is than centrifuged for 20 minutes at 10,000g and the absorbed antiserum in the supernatant is collected.

Example 3

Production of monoclonal anti GP 70

A. Immunization and Somatic Cell Hybridization

Female Balb/cJ mice (Hadassah Hospital Farm; 6-8 weeks old) were immunized intraperitoneally with 5µg of purified GP 70 in 0.2 ml of phosphate buffered saline at 30 day intervals. Four days after the third immunization, spleens were removed from the mice, and a single cell suspencion was made by pressing the tissue through a stainless steel mesh.

Cell fusion was carried out according to the procedure developed by Kohler and Milstein (ibid). One hundred million splenocytes were fused in 0.5 ml of a fusion medium comprising 35% polyethylene glycol (PEG 1000) and 5% dimethylsulfoxide in RPMI 1640 medium (Gibco, Grand Island, NY) with two hundred million 653 myeloma cells supplied by Dr R. Laskov, Hadassah Medical School, Jerusalem. These myeloma cells are non secretor cells.

## B. Selelction and Growth of Hybridoma

After cell fusion, cells were cultured in HAT medium (Hypoxanthine, aminopterin, and thymidine) at $37^\circ$ C with 5% $CO_2$ in a humid atmosphere. Two weeks later, 25 to 50 $\mu$l of supernatant from cultures containing hybridomas were added to a pellet of one million Daudi cells. Detection of mouse hybridoma antibodies binding to these cells was determined by indirect immunofluorescence. Briefly, cells incubated with culture suppernatants were stained with fluorescinated goat-anti-mouse IgG (G/M FITC) (Bio-Yeda Laboratories, Rehovot, Israel) and the fluorescent antibody-coated cells were analysed by Zeiss

0179450

fluorescence microscope and subsequently analyzed on the fluorescence activated cell sorter (FACS) Becton Dickinson, as described in example 5. Hybridoma cultures containing antibodies reacting specifically with Daudi cells were selected and cloned. Subsequently, the clones were transferred intraperitoneally by injecting one million cells of a given clone (0.2 ml volume) into Balb/cJ mice primed with 2,6,10,14 -tetramethylpentadecane, sold by Aldrich Chemical Company under the name of Pristane. The malignant ascites from these mice were then used to characterize lymphocytes as described below in example 4. The subject hybridoma antibody to GP 70 was demonstrated by standard techniques to be of IgM class.

One of the obtained hybridomas was hybridoma ($B_2$) (CNCM          ).

## Example 4

## Characterization of anti GP 70 Reactivity

## A. Isolation of Lymphocyte Populations

Human peripheral blood mononuclear cells were isolated from healthy volunteer donors or leukemia-lymphoma patients by Ficoll-Hypaque density gradient centrifugation (Pharmacia Fine Chemicals, Sweden) following the technique of Boyum, Scand.J. Clin. Lab. Invest. 21 (Suppl. 97): 77, 1968. Normal human macrophages were obtained from the mononuclear population by adherence to polystyrene. Thus, mononuclear cells were resuspended in final culture media (RPMI 1640, 2.5 mM HEPES [4-(2-hydroxyethyl)-1-piperazinepropane sulfonic acid] buffer, 0.5% sodium bicarbonate, 200 mM L-glutamine, and 1% penicillin-streptomycin, supplemented with 20% heat-activated human A8 serum at a concentration of two million cells and incubated in plalstic petri dishes (100 x 20 mm) (Falcon Tissue Culture Dish; Falcon, Oxfard, CA) at 37° C overnight. After extensive washing to remove non-adherent cells, the adhertent population was detached by brisk washing with cold serum-free medium containing 2.5 mM EDTA and occasional scraping with the rubber tip of a disposable syringe plunger. Greater than 85% of the cell population was capable of ingesting latex particles and had morphologic characteristics of monocytes by Wright-Giemsa staining.

## B. Normal Lymph nodes and spleen

Normal human spleen was obtained from splenetomized patients and lymph nodes from patients with suspected breast cancer. Only negative lymph nodes were used. Freshly obtained portions of the organs were immediately placed in 5% fetal calf serum in medium 199 (Gibco), finely minced with forceps and scissors, and subsequently made into single cell suspensions by being pressed through wire mesh. The cells were next layered over Ficoll-Hypaque spun and washed as previously described in section A above. The mononuclear fraction so obtained contained 95% viable cells.

## C. Blood cells, tonsils and bone marrow.

Blood cells were collected from veins of healthy volunteers or leukemia patients. Heparin was used as an anti coagulant. The mononuclear fraction and platelet fraction were obtained from the interphase of a Ficoll-Hypaque (Farmacia Fine Chemicals, Sweden) gradient. Granulocytes and erythrocytes were collected from the pellet of the gradient. Granulocytes were obtained from pellet fraction following hemolysis of erythrocytes in hypotonic PBS (1/10 tonicity) followed by a restoration of isotonicity with concentrated PBS. All cell types thus obtained were washed three times with PBS and immunofluorescence test was performed on the same day.

Tonsils were obtained from Dr. R. Feinmesser and bone marrow from Dr. S. Slavin both from Hadassah Hospital, Jerusalem. Tissues were macerated and single cell suspension formed was washed 3 times with PBS and layered on a Ficoll-Hypaque gradient. The interphase fraction was collelcted, washed and Kept for immunofluorescence studies.

## D. Cell Lines.

Epstein-Barr Virus (EBV) transformed B cell lines from nine normal individuals and 23 BL cell lines from BL patients were obtained from Dr. H. Ben Bassat, Department of Experimental Surgery, Hadassah Hospital, Jerusalem and from Dr. G. Klein, Karolinska Institute, Stockholm.

## Example 5

## Cytofluorographic Analysis and Cell separation

Cytofluorographic analysis of all cell population was performed by indirect immunofluorescence with fluorescein-cojugated goat-anti-mouse IgG (G/M FITC) (Bio-Yeda Laboratories) on fluorescence activated cell sorter (FACS) (Becton Dickenson Instruments). In brief, one million cells were treated with 0.10 ml anti GP 70 at 1:100 dilution for polyclonal antibodies and at 1:800 dilution for monoclonal antibodies, incubated at $25^{O}C$ for 30 minutes, and washed twice. The cells were then reacted with 0.10 ml of a 1:20 dilution G/M or G/R FITC at $25^{O}C$ for 30 minutes,

centrifuged and washed three times. These cells were then analyzed both by fluorescent microscope and by the FACS and the percent stained cells or the intensity of fluorescence per cell were examined. Background staining was obtained in the case of monoclonal antibodies by substituting a 0.10 ml aliquot of 1:800 ascites from a Balb/cJ mouse intraperitoneally immunized with a non-producing hybrid clone. In the case of polyclonal antibody Normal Rabbit Serum (NRS) at 1:100 dilution was used for background screening.

Example 6

Immunofluorescence Staining

Anti GP 70 antiserum at a dilution of 1:100-1:200 is used to stain cells in an indirect double antibody fluorescent stain using FITC - goat anti rabbit Ig (Bio-Yeda, Rehovot, Israel) as second antibody. Staining procedure is carried out as follows: washed cell suspension (1-5 x $10^6$/0.1 ml) are incubated with 0.1 ml of test serum (1:100 dilution) in the presence of 0.1% sodium azide for 30 minutes at room temperature. Unbound material is washed out with PBS and incubation repeated with second antibody (1:20 dilution). Unbound second antibody is washed out with PBS-azide and cells are monitored within 1-2 hours in a fluorescence microscope (Zeiss).

Example 7

Analysis of protein by sodium dodecyl sulfate polyacrylamide gel electrophoresis (SDS-PAGE) and autoradiography

Samples of 0.05-0.1 mg were diluted in sample buffer containing 1% SDS, 0.1M DTT, 50% glycerol in 0.5M Tris-phosphate buffer pH,6.5 and heated for 5 minutes at 96°C. After cooling, the samples were applied onto a gradient slab gel 9%-20% acrylamide, and electrophoresed according to Laemmli as outlined in Nature (Lond) 227, 680-684, 1970, at a constant voltage of 140V for 3 hours. The gels were stained for 30 minutes in a solution of 0.25% Coomassie blue (R250-Biorad) in 50% methanol/10% acetic acid. Destaining was accomplished in a solution of 10% acetic acid. Gels with radiolabelled proteins were dried and exposed to autoradiography at 70°C with MRF 31 x-ray film (Agfa, Geveart) in Kodak regular type intensifying screen. Exposure of 3-6 days was adequate to detect 10,000-50,000 cpm.

The invention is further illustrated by the following drawings:

Figure 1 is a schematic representnaion of the isolation and purification procedure of GP 70 from BL sera as described above in Example 1.

Although only a specific isolation and purification procedure of GP 70 from BL sera is described it should be contemplated that the present invention extends also to other affinity chromotography methods which yield similar purified antigen and to other natural sources of GP 70 such as CLL sera.

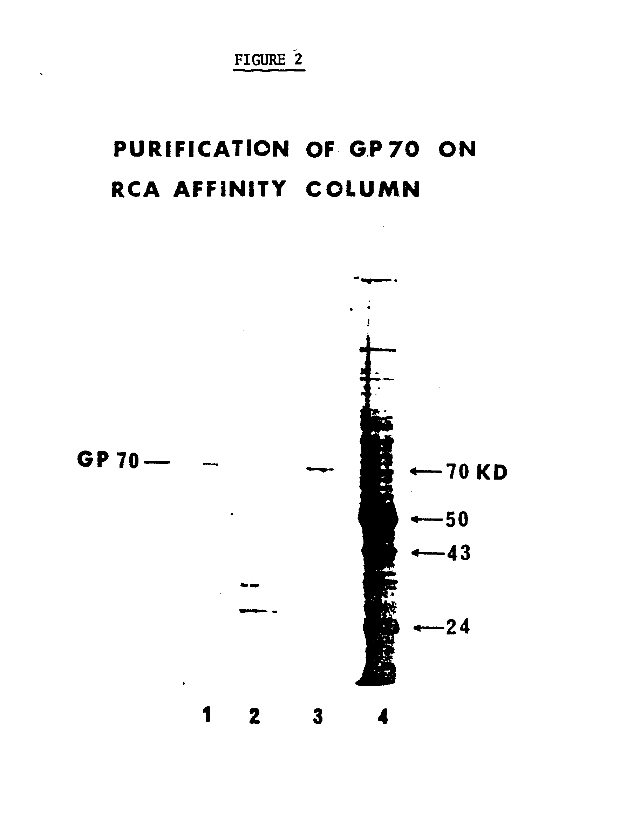

Figure 2 shows the purification of GP 70 on RCA affinity column, as exhibited by analysis on SDS-PAGE described in Example 7. Lane 1 represents material from stage III of Figure 1; Lane 2 represents material passed through RCA column and material bound to RCA and eluted with specific suger. Precolumn material is represented by lane 3. For comparison, cell membrane extract from Daudi cells containino GP 70 prepared as outlined by Gazitt et al in cancer Res 41, 1184-1186, 1981, is represented in lane 4. As shown in figure 2 the GP 70 band found in BL sera comigrates with similar band of 70 KD found in Daubi cell membrane.

Figure 3 shows analytical results for anti GP 70 precipitated proteins from $^{35}$S - Methionine labled Daudi Calls cell membrane.

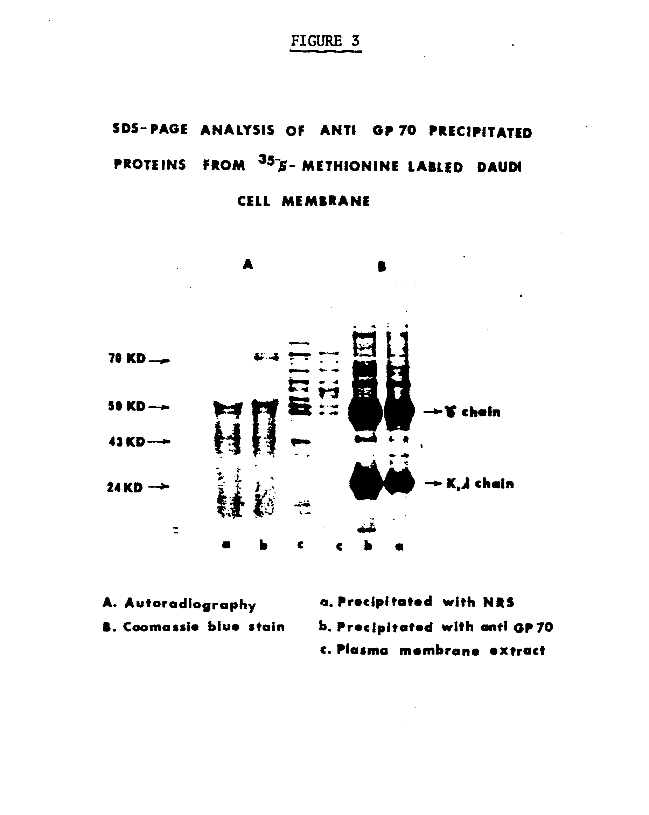

Daudi cells ($10^8$/20 ml) were incubated with 1,mci of $^{35}$S - Methionine (Amersham, 800 ri/mmole) in RPMI Medium without methionine (GIBCO, Grand Island, N.Y.) for 12 hours. Plasma membrane fraction was purified as described by Gazitt et al, ibid, and 2 x $10^6$ cpm were reacted with 10 μl of antibody or normal rabbit serum (NRS). Immunecomplexes were precipitated with goat and rabbit Ig at point of equivalence as described by Gazitt et al in Int. J. Cancer 29, 645-651, 1982. Washed precipitates were analysed on SDS-PAGE and proteins were visualized by coomassi blue stain (0) or following autoradiography (A) as described by Gazitt et al, ibid. Lane a in Figure 3 represents material precipitated with NRS, lane b represents precipitation with anti GP 70, and lane c represents purified plasma membrane fraction.

Figure 3 shows that the 70 KD band is present in the immune precipitate of Daudi cell membrance with immune serum (lane b) but not with non-immune serum (lane a), section A.

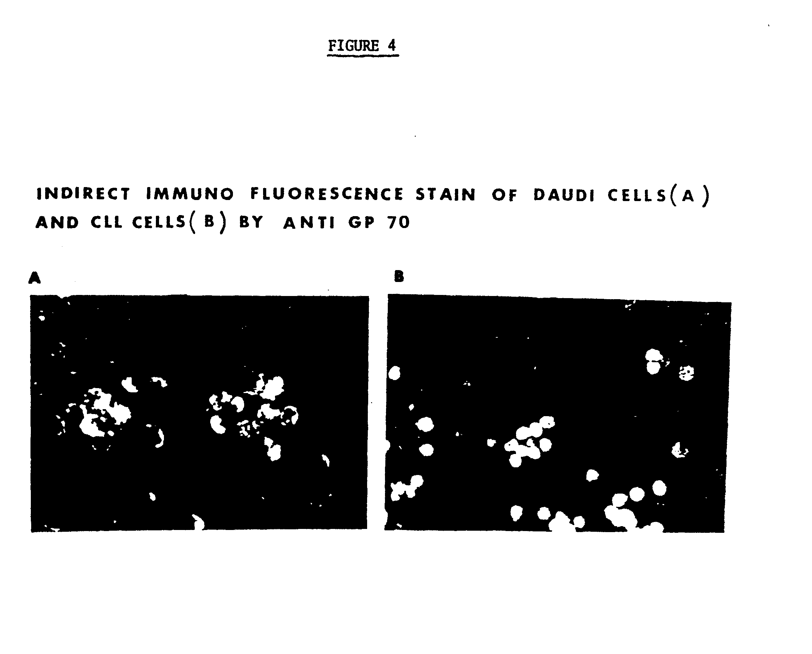

Figure 4 shows fluorescence stain of Daudi cells and CLL cells with anti GP 70 antibodies.

One million cells were washed three times with PBS and antibodies were added (0.1 ml of 1:100 dilution). Mixture was Incubated for 30 minutes at 25°C and unbound antibodies were washed out with PBS containing sodium azide (0.1% final concentration). FITC- goat anti rabbit Ig (Bio-Yeda, Rehovot, Israel; 0.1 ml of 1:20 dilution) was added as described above. Unbound second antibody was washed out and fluorescent cells visualized under the Zeiss fluorescent microscope. Photographs were taken with Kodak 200 ASA film. Magnification is 400 times. As shown in Figure 4 the fluorescence intensity of the majority of both Daudi cells (A) as well as of CLL cells (B) labled with anti GP 70 is very high. At the same time same cells, stained with non immune serum (NRS), showed no fluorescence at all.

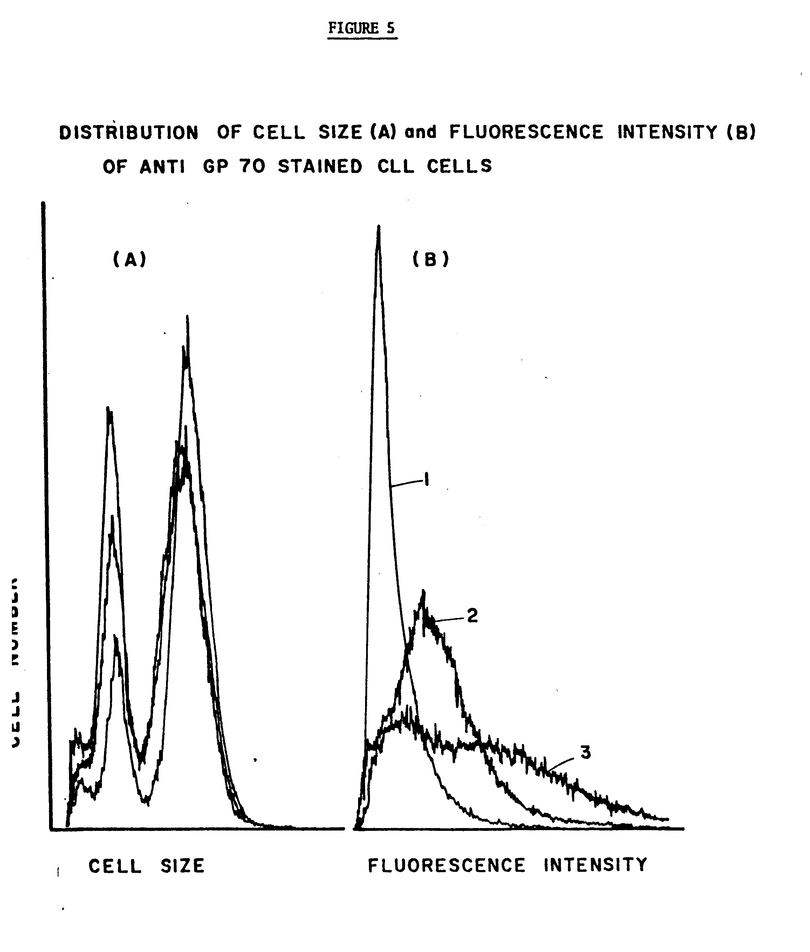

Figure 5 shows the distribution of cell size and fluorescence intensity of anti GP 70 stained CLL cells.

CLL cells were obtained from the peripheral blood of a typical CLL patient under conventional treatment at Hadassah Hospital, Jerusalem. The Ficcoll-Hypaque mononuclear fraction was isolated and used for fluorescence stain as described above. Thirty thousand cells were analysed in the fluorescence activbated cell sorter (FACS, Becton Dickinson, CA) at 500V. The distribution of cell size by light scatter and of the fluorescence intensity

per cell as recorded are represented in A and B respectively.1
represents NRS (normal rabbit serum) which is a negative control;
2 refers to SmIg cells stained with rabbit anti human Ig (0.1 ml
of 1:100 dilution) and 3 to cells reacted with anti GP 70 (0.1 ml
of 1:100 dilution). The difference between the patterns obtained
with the antibodies represented in Figure 5, indicates that those
antibodies recognize different antigens.

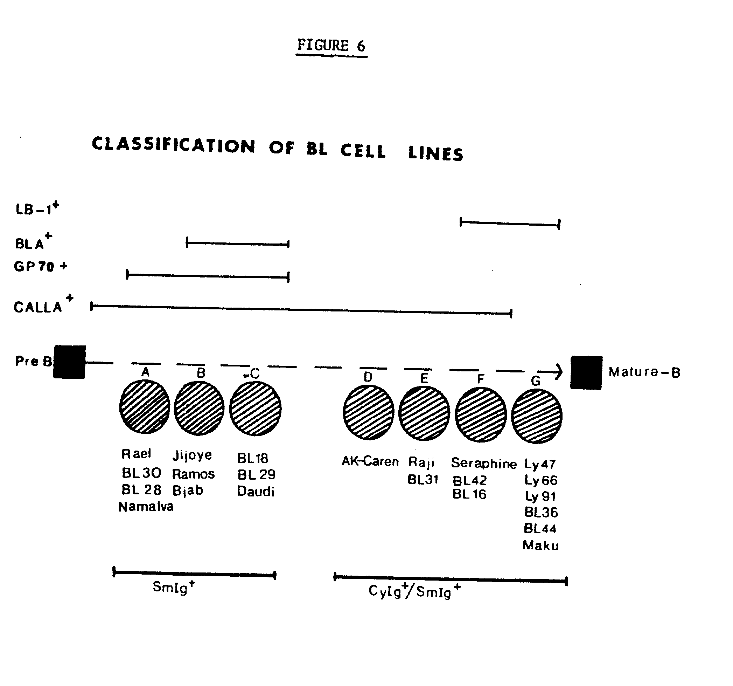

Figure 6 shows a schematic representation of a classification
of BL cell lines. All cell lines used were obtained from Dr. G.
Klein (Karolinska institute, Stockholm).One million cells were
cultured by conventional methodology, prepared for fluorescence
stain and stained as described above. Data on LB-1 stain were
taken from Klein et al Int. J. Cancer 130, 1985-1989, 1983 and
data on BLA and CALLA stain were taken from Ehlin Henriksson et
al, 1984, in press. As shown in Figure 6 the stage of
differentiation of BL cells recognized by anti GP 70 is very
narrow and occurs in early immature BL cells. Thus anti GP 70 is
advantageous to other known markers which recognize a much wider
range of differentiation stages and therefore as also outlined in
table VII, all those other known markers have a wider recognition
of different leukemias and lymphomas.

The pattern of binding of GP 70 to various cell lines is shown in tables I-V. Table I shows that anti GP 70 binds effectively to B-cell lines originated from Burkitt's lymphoma patients. Thus about 60 percent of BL cancer cell lines were stained, in particular early B cells containing surface membrane Ig, while more differentiated lines containing cytoplasmic Ig were negative. On the other hand, none of the ten lymphoblastoid cell lines (non-malignant B-cells) listed in table II is positive to anti GP 70, indicating specifity to cancer cells. Moreover, cell lines derived from T-ALL and Hodgkins disease patients were all negative as shown in table III, indicating specifity to B-cells.

Similar results are found when malignant cells of myelocytic lineage or ephitelial cell lines derived from breast, colon, lung and hepatoma cell lines are tested as shown in table IV. Furthermore, as shown in table V, all peripheral blood cells from normal donors do not bind anti GP 70.

Examination of the lymphocyte fraction of 139 different leukemia patients, 40 different lymphoma patients and 263 healthy individuals by immunofluorescence staining as described above, showes as summarized in table VI that GP 70 is present only on cell from BL and CLL patients. None of the cells from healthy individuals reacted with anti GP 70. Moreover, all of the cells of leukemia and lymphoma patients other than CLL and BL were negative to GP 70.

Table VII compares between GP 70 and other B-cell marker known from the prior art.

Table I:  Fluorescence stain of BL cell lines with anti GP70

| Cell line | No. tests | GP70 (% positive) |
|---|---|---|
| Jijoye | 5 | 61 ± 10 |
| PyHK-1 | 5 | 0 |
| Namalva | 7 | 51 ± 12 |
| Daudi | 7 | 62 ± 12 |
| Raji | 5 | 2 ± 1 |
| Raal | 3 | 33 ± 1 |
| Ujab | 8 | 48 ± 11 |
| Rxros | 3 | 86 ± 4 |
| *Rxros/Hrik | 4 | 94 ± 1 |
| *Rxros/EHR-A | 5 | 77 ± 10 |
| *Rxros/AW | 5 | 75 ± 9 |
| **Rxros/E-95-0 | 5 | 76 ± 8 |
| BL 16 | 8 | 27 ± 9 |
| BL 18 | 2 | 60 ± 6 |
| BL 29 | 4 | 82 ± 6 |
| BL 30 | 5 | 68 ± 8 |
| BL 31 | 5 | 1 ± 0.5 |
| BL 36 | 3 | 0 |
| BL 42 | 5 | 15 ± 7 |
| BL 44 | 3 | 0 |
| LY 47 | 3 | 0 |
| LY 66 | 4 | 9 ± 3 |
| LY 91 | 3 | 0 |
| AK-Caren | 3 | 0 |
| Seraphine | 3 | 3 ± 3 |
| HaKu | 3 | 0 |

* PJHR-1 EBV Converted ;  ** B-95-EBV Converted.

Note:  Cell lines were cultured by routine methodology in RPMI 1640 + 20% fetal calf serum (obtained from Grand Island Biological Corporation, Grand Island, N.Y.) as described by Ben Bassat et al. in Cancer 40, 1481-1491, 1977.  Log phase cells were prepared for staining and stained as described in Example 6.

Table II: Fluorescence stain of lymphoblastoid cell lines
with anti GP70

| Cell line | No. tests | GP70 (% positive) |
|-----------|-----------|-------------------|
| MP-B1 | 3 | 1 + 1 |
| ES-B1 | 3 | 2 + 1 |
| AK 11,12 | 3 | 9 + 8 |
| SPIM-160 | 4 | 10 + 5 |
| JARC 167 | 3 | 0 |
| JARC 174 | 4 | 5 + 3 |
| JARC 176A | 3 | 0 |
| Lans Sternas | 3 | 0 |
| NAD-20 | 3 | 7 + 7 |
| Jarc 99 | 3 | 0 |

Legend as described in Table 1.

Table III:  Immunofluorescence stain of T-Leukemic
            cells with anti GP70

| Cell line | Binding % | | Origin of cell line |
| --- | --- | --- | --- |
| | GP70 | NRS | |
| HPb-ALL | < 2 | < 2 | T-ALL patient |
| Peer | < 2 | < 2 | T-ALL patient |
| Farage | < 2 | < 2 | T-ALL patient |
| Molt-4 | < 2 | < 2 | T-ALL patient |
| Sill-ALL | < 2 | < 2 | T-ALL patient |
| HD-MAR | < 2 | < 2 | Hodgkin's disease |

T-ALL = Acute Lymphoblastic Leukemia of T-Cell type.

Legends as in Table I.

TABLE IV: Binding of anti GP70 to cancer cell lines of various tissues

| Cell line | Binding % | | Origin of cell line |
|---|---|---|---|
| | GP70 | NRS | |
| M 7 | < 2 | < 2 | CML - patient |
| M 4 | < 2 | < 2 | CML - patient |
| HL-60 | < 2 | < 2 | Acute promyelocytic Leukemia |
| GDM-1 | < 2 | < 2 | Myeloid Leukemia |
| REH | < 2 | < 2 | N - ALL |
| KM-3 | < 2 | < 2 | N - ALL |
| HU | < 2 | < 2 | Epitheial cell line |
| 1063 | < 2 | < 2 | Ovarian carcinoma |
| MDA | < 2 | < 2 | Breast cancer |
| Fogh | < 2 | < 2 | Hepatoma |
| Ewing | < 2 | < 2 | Colon carcinoma |

CML = Chronic Myelogenous Leukemia

Legend as in Table 1.

Table V: Binding of anti GP70 antibodies to normal peripheral blood cells

| Blood Cell Type | Binding (%) | |
|---|---|---|
| | Anti GP40 | NRS |
| Peripheral Blood  Lymphocytes | < 2 | < 2 |
| B - Lymphocytes | < 2 | < 2 |
| T - Lymphocytes | < 2 | < 2 |
| Granulocytes | < 2 | < 2 |
| Erythnocytes | < 2 | < 2 |
| Platelets | < 2 | < 2 |
| Tonsils cells | < 2 | < 2 |
| Bone marrow cells | 10-20 | 10-20 |

Cells were isolated as described in Example 8 and stained as described in Example 6.

Table VI: Clinical evaluation of anti GP70 antibody

| type of cancer | No. patients | No. positive | % positive | Remarks |
|---|---|---|---|---|
| *ALL | 48 | 0 | 0 | All ALL were CALLA+ |
| CLL | 63 | 55 | 87 | 5 out of the 8 negative patients were in the acute stage. |
| HCL | 4 | 0 | 0 | |
| AML | 12 | 0 | 0 | |
| CML | 10 | 0 | 0 | |
| MM | 2 | 0 | 0 | |
| BL | 8 | 6 | 75 | Biopsies were taken from tumors. |
| HL | 6 | 0 | 0 | Biopsies were taken from bone marrow and lymph nodes |
| NHL | 16 | 0 | 0 | |
| Controls-Normals | 260 | 0 | 0 | Blood bank donors |
| Controls IM | 3 | 0 | 0 | Patients were sampled at various stages of disease |

*ALL - Acute lymphoblastic leukemia; CLL - Chronic lymphocytic leukemia; HCL - Hairy cell leukemia; AML - Acute myelogenous leukemia; CML - Chronic myelogenous leukemia; MM - Multiple myeloma; BL - Burkitt's lymphoma; HL - Hodjkin's lymphoma; NHL - non-Hodjkin's lymphoma; IM - Infectious mononucleosis.

TABLE VII: Comparison between GP70 and other B-cell markers

| B-cell marker | Molecular Weight (M.W) | Specificity | Reactivity with BL cell lines | Remarks |
|---|---|---|---|---|
| B1 | 30 KD | ALL, CLL and all lymphomas | most BL cell lines are stained | stains CML at blast crisis. |
| B2 | 140 KD | ALL, CLL and all lymphomas | stains more differentiated BL cell lines | stains also lymphoblastoid cell lines and mature B-cells. |
| B4 | >120 KD | pre B-ALL and normal B cells | ? | "pan B" stains all stages of B-cell differentiation |
| a)BLA | A glycolipid of small M.W. | some CLL and BL most lymphomas. | stains immature BL cell lines. | |
| a)CALLA | 100KD | C-ALL | most BL cell lines except for mature cIg containing BL cells. | stains also cells from other tissues (i.e epithelial) |
| a)LB-1 | ? | stains mature normal β cells and most ALL and CLL | stains mature BL cell lines. | a normal B cell marker. |
| BA-1 | ? | stains mature normal B cells and most ALL and CLL | stains mature BL cell lines. | a normal B cell marker. |
| a)GP70 | 70KD | stains all CLL and BL. | stains only early and immature BL cell lines. | does not react with lymphoma cells except for bL. |

a) For comparison see also Figure 5.

CLAIMS

1. An antibody which reacts with B cells derived from BL and CLL patients and does not react with B cells and T cells derived from normal individuals or B cells and T cells derived from patients with cancer disorders other than BL or CLL.

2. An antibody according to claim 1 which does not react with normal peripheral blood lymphocytes, red blood cells, platelets, granulocytes, non-cancerous B-lymphoblastoid, B or T cells stimulated by lectin mitogen, T-All, AML, CML cell lines, T cell lines and cell lines derived from breast, ovary, liver and lung cancer patients.

3. A glycoprotein of 70 KD in substantially pure form, referred to as GP 70 hereinabove, which reacts with the antibody of claim 1.

4. A method of isolation and purification of GP 70 according to claim 3, which comprises subjecting the serum of BL or CLL patients to a series of affinity chromatography separation.

5. A method of isolation and purification of GP 70 according to claim 4, which comprises the following steps:
   (a) removing from sera the immunoglobulin fraction by column chromatography on DEAE-52 cellulose and eluting the GP 70 enriched fraction with 50 mM phosphate buffer pH 7.8;
   (b) binding the immunoglobuline-free fraction to a concanavalin A sepharose column;
   (c) eluting the bound fraction in (b) by methyl mannoside;
   (d) passing the fraction so obtained on a Ricin Communis Agglutinin (RCA) sepharose column and collecting the non-bound fraction.

6. A polycolonal antibody of claim 1 prepared by the method which comprises the steps of:
   (a) immunizing animals with GP 70;
   (b) bleeding said animals to collect antisera;
   (c) absorbing said antisera on tissue capable of binding all normal human antibodies if there are any;

(d) centrifuging the suspension; and

(e) recovering the antibody from the supernatant.


7.   A method of preparing a polyclonal antibody according to claim 1 which comprises the steps of:

(a) immunizing animals with GP 70;

(b) bleeding said animals to collect antisera;

(c) absorbing said antisera on tissue capable of binding all normal human antibodies if there are any;

(d) centrifuging the suspension; and

(e) recovering the antibody from the supernatant.


8.   A monoclonal antibody of claim 1 prepared by the method which comprises the following steps:

(a) immunizing mice with purified GP 70;

(b) removing the spleen from said mice and making a suspension of spleen cells;

(c) fusing said spleen cells with mouse myeloma cells in the presence of a fusion promoter;

(d) diluting and culturing the fused cells in
separate wells in a medium which will not
support the unfused myeloma cells;

(e) evaluating the supernatant in each well
containing a hybridoma for the presence
of the desired antibody;

(f) selection and cloning hybridomas producing
the desired antibody; and

(g) recovering the antibody from the supernatant
above said clones.


9. A monoclonal antibody of claim 1 which is produced from
hybridoma formed by fusion of 653 myeloma cells and spleen
cells from a Balb/CJ mouse previously immunized with GP 70.


10. A monoclonal antibody of claim 1 which is produced from a
hybridoma having the identifying characteristics of CNCM
number.


11. An IgG monoclonal-antibody-producing hybridoma formed by
fusion on spleen cells from mouse previously immunized with
GP 70 and cells from mouse myeloma line.

12. The hybridoma of claim 11 formed by fusion of spleen cells from Balb/CJ mouse previously immunized with GP 70 and 653 myeloma cells.

13. A method of diagnosis of BL or CLL which comprises reacting serum of patient or other fraction of body fluid of patient with an antibody of claim 1 and determining the immune complex with a known immunological test method.

14. A method of diagnosis for BL or CLL which comprises the steps of:

    (a) obtaining a mononuclear fraction of lymphocytes from patient's blood to be tetsted;

    (b) adding to said fraction an antibody of claim 1;

    (c) precipitating said antibody by a second labled antibody directed against the first antibody; and

    (d) detecting the labled antibody in the precipitate for a positive result.

15. A method of diagnosis according to claim 14 wherein the labled second antibody is labled by fluorescein.

16. A pharmaceutical composition comprising as active material a medicament directed against BL and CLL bound to an antibody of claim 1.

17. A composition comprising as active material an antibody of claim 1 bound to ricin toxin.

18. A method of treating BL or CLL comprising administering to a patient an effective amount of a pharmaceutical composition comprising as active material a medicament directed against BL and CLL bound to an antibody of claim 1.

19. A method of treating BL or CLL comprising administering to a patient an effective amount of a composition comprising as active material an antibody of claim 1 bound to ricin toxin.

20. A method of treating BL or CLL comprising administering to a patient an effective amount of the antibody of claim 1.

21. A method of in-vivo removal of CLL or BL cells from plasma by means of techniques related to hemoperfusion comprising subjecting the plasma of a patient to a selective binding of tumor cells to anti GP 70 attached to a solid support.

22. A method of an in-vivo imaging of BL tumors by means of antibody of claim 1 labled by radioactive agent.

23. A pharmaceutical composition comprising as active material an antibody of claim 1.

YISSUM RESEARCH DEVELOPMENT COMPANY
OF THE HEBREW UNIVERSITY, JERUSALEM
Our Ref: U 114 EP

1/6

## FIGURE 1

**ISOLATION AND PURIFICATION OF GP 70**

Stage I

Stage II

Stage III

YISSUM RESEARCH DEVELOPMENT COMPANY
OF THE HEBREW UNIVERSITY, JERUSALEM

0179450

SIX SHEETS
SHEET No. 2

2/6

FIGURE 2

# PURIFICATION OF G.P 70 ON RCA AFFINITY COLUMN

GP 70 —

70 KD

50

43

24

1    2    3    4

YISSUM RESEARCH DEVELOPMENT COMPANY
OF THE HEBREW UNIVERSITY, JERUSALEM

0179450
SIX SHEETS
SHEET No. 3

3/6

FIGURE 3

SDS-PAGE ANALYSIS OF ANTI GP 70 PRECIPITATED

PROTEINS FROM $^{35}S$- METHIONINE LABLED DAUDI

CELL MEMBRANE

A. Autoradiography
B. Coomassie blue stain

a. Precipitated with NRS
b. Precipitated with anti GP 70
c. Plasma membrane extract

YISSUM RESEARCH DEVELOPMENT COMPANY
OF THE HEBREW UNIVERSITY, JERUSALEM

0179450
SIX SHEETS
SHEET No. 4

4/6

FIGURE 4

INDIRECT IMMUNO FLUORESCENCE STAIN OF DAUDI CELLS(A) AND CLL CELLS(B) BY ANTI GP 70

0179450

YISSUM RESEARCH DEVELOPMENT COMPANY
OF THE HEBREW UNIVERSITY, JERUSALEM

SIX SHEETS
SHEET No. 5

5/6

FIGURE 5

DISTRIBUTION OF CELL SIZE (A) and FLUORESCENCE INTENSITY (B)
OF ANTI GP 70 STAINED CLL CELLS

6/6

FIGURE 6

## CLASSIFICATION OF BL CELL LINES